(19) Europäisches Patentamt
European Patent Office
Office européen des brevets

(11) EP 2 074 153 B1

(12) **EUROPÄISCHE PATENTSCHRIFT**

(45) Veröffentlichungstag und Bekanntmachung des Hinweises auf die Patenterteilung:
**09.09.2015 Patentblatt 2015/37**

(21) Anmeldenummer: **07820241.3**

(22) Anmeldetag: **17.09.2007**

(51) Int Cl.:
***C08F 6/10*** *(2006.01)* ***A61L 15/22*** *(2006.01)*
***C08F 20/06*** *(2006.01)* ***F26B 17/04*** *(2006.01)*
***F26B 21/06*** *(2006.01)* ***C08J 3/12*** *(2006.01)*
***A61L 15/60*** *(2006.01)* ***C08F 220/06*** *(2006.01)*
***C08F 222/10*** *(2006.01)* *C08L 33/02* *(2006.01)*

(86) Internationale Anmeldenummer:
**PCT/EP2007/059759**

(87) Internationale Veröffentlichungsnummer:
**WO 2008/034786 (27.03.2008 Gazette 2008/13)**

(54) **VERFAHREN ZUR HERSTELLUNG FARBSTABILER WASSERABSORBIERENDER POLYMERPARTIKEL MIT NIEDRIGEN NEUTRALISATIONSGRAD**

PROCESS FOR PREPARING COLOR-STABLE WATER-ABSORBING POLYMER PARTICLES WITH A LOW DEGREE OF NEUTRALIZATION

PROCÉDÉ DE FABRICATION DE PARTICULES POLYMÈRES ABSORBANT L'EAU ET STABLES AUX COLORANTS, AVEC UN DEGRÉ DE NEUTRALISATION RÉDUIT

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **19.09.2006 EP 06120881**

(43) Veröffentlichungstag der Anmeldung:
**01.07.2009 Patentblatt 2009/27**

(73) Patentinhaber: **BASF SE**
**67056 Ludwigshafen (DE)**

(72) Erfinder:
- **FUNK, Rüdiger**
  **65527 Niedernhausen (DE)**
- **BRAIG,Volker**
  **69469 Weinheim-Lützelsachsen (DE)**

(56) Entgegenhaltungen:
**EP-A- 0 238 050**
**EP-A- 1 512 417**
**WO-A-2006/100300**
**WO-A1-2005/103119**
**US-A- 5 668 252**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 074 153 B1

Printed by Jouve, 75001 PARIS (FR)

## Beschreibung

Die vorliegende Erfindung betrifft ein Verfahren zur Herstellung wasserabsorbierender Polymerpartikel durch Polymerisation einer Monomerlösung, enthaltend mindestens ein ethylenisch ungesättigtes, säuregruppentragendes Monomer, das zu weniger als 55 mol-% neutralisiert ist, und Trocknung des erhaltenen Polymergels mittels eines erwärmten Gasstromes, wobei die Trocknung in mindestens zwei Temperaturzonen durchgeführt wird.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung der wasserabsorbierenden Polymere wird beispielsweise in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seiten 73 bis 103, beschrieben.

Üblicherweise wird nach der Polymerisation ein wässriges Polymergel erhalten, das getrocknet werden muss. Die Trocknung des Polymergels wird ebenfalls in der Monographie "Modern Superabsorbent Polymer Technology", F.L. Buchholz und A.T. Graham, Wiley-VCH, 1998, Seiten 87 bis 93, offenbart.

EP 289 338 A2 beschreibt ein Verfahren zur Trocknung von Polymergelen, wobei der zum Trocknen verwendete Gasstrom zumindest anfangs einen Taupunkt von 50 bis 100 °C aufweist.

EP 1 002 806 A1 offenbart ein Verfahren zur Herstellung wasserabsorbierender Polymere, wobei ein Polymergel in mindestens drei Abschnitten getrocknet wird.

Die ältere deutsche Patentanmeldung mit dem Aktenzeichen 102005014291.5 beschreibt ein Trocknungsverfahren mit einem Temperaturprofil.

Aufgabe der vorliegenden Erfindung war die Bereitstellung eines verbesserten Verfahrens zur Herstellung wasserabsorbierender Polymerpartikel mit niedrigem Neutralisationsgrad, insbesondere eine verbesserte Trocknung der während des Verfahrens anfallenden wässrigen Polymergele.

Eine weitere Aufgabe der vorliegenden Erfindung war die Bereitstellung von farbstabilen wasserabsorbierenden Polymerpartikeln mit niedrigem Neutralisationsgrad, d. h. von Polymerpartikeln, die bei längerer Lagerung nicht zur Vergilbung neigen.

Gelöst wurde die Aufgabe durch Verfahren zur Herstellung wasserabsorbierender Polymerpartikel durch Polymerisation einer Monomerlösung, enthaltend

a) mindestens ein ethylenisch ungesättigtes, säuregruppentragendes Monomer, das zu weniger als 55 mol-% neutralisiert ist,
b) mindestens einen Vernetzer,
c) wahlweise ein oder mehrere mit den unter a) genannten Monomeren copolymerisierbare ethylenisch und/oder allylisch ungesättigte Monomere und
d) wahlweise ein oder mehrere wasserlösliche Polymere,

wobei die Monomerlösung polymerisiert und das erhaltene Polymergel mittels eines erwärmten Gasstromes getrocknet wird, dadurch gekennzeichnet, dass die Trocknung in mindestens zwei Temperaturzonen durchgeführt wird, wobei die Gaseingangstemperaturen der mindestens zwei Temperaturzonen die Bedingung $T_n$ größer $T_{n+a}$ erfüllen, wobei die Indizes n und a jeweils eine ganze Zahl größer 0 bedeuten, sowie die Gaseingangstemperatur in der Temperaturzone $T_n$ weniger als 175 °C und die Temperaturdifferenz der Gaseingangstemperaturen der Temperaturzonen $T_n$ und $T_{n+a}$ mehr als 10 °C beträgt und das Polymergel nach der Trocknung einen Wassergehalt von 3 bis 10 Gew.-% aufweist.

Die Indizes geben die zeitliche und/oder räumliche Abfolge der Temperaturzonen an, die das zu trocknende Gut in aufsteigender Abfolge durchläuft. Eine Temperaturzone ist ein Bereich, in dem die Gaseingangstemperatur unabhängig eingestellt werden kann.

Der Erfindung liegt die Erkenntnis zugrunde, dass sich Polymergele bei Trocknung je nach Neutralisationsgrad völlig unterschiedlich verhalten und dass die Trockenbedingungen des Polymergels einen entscheidenden Einfluss auf die Farbstabilität der hergestellten wasserabsorbierenden Polymerpartikel haben.

Die Gaseingangstemperatur $T_n$ beträgt vorzugsweise mindestens 120 °C, bevorzugt mindestens 130 °C, besonders bevorzugt mindestens 140 °C, ganz besonders bevorzugt mindestens 150 °C, und vorzugsweise weniger als 173 °C, bevorzugt weniger als 171 °C, besonders bevorzugt weniger als 169 °C, ganz besonders bevorzugt weniger als 167 °C.

Die Gaseingangstemperatur $T_{n+a}$ beträgt vorzugsweise mindestens 100 °C, bevorzugt mindestens 110 °C, besonders bevorzugt mindestens 120 °C, ganz besonders bevorzugt mindestens 130 °C, und vorzugsweise weniger

als 160 °C, bevorzugt weniger als 158 °C, besonders bevorzugt weniger als 156 °C, ganz besonders bevorzugt weniger als 154 °C.

Der Temperaturdifferenz der Gaseingangstemperaturen $T_n$ und $T_{n+a}$ beträgt vorzugsweise mehr als 11 °C, bevorzugt mehr als 12 °C, besonders bevorzugt mehr als 13 °C, ganz besonders bevorzugt mehr als 14 °C, und vorzugsweise weniger als 50 °C, bevorzugt weniger als 40 °C, besonders bevorzugt weniger als 35 °C, ganz besonders bevorzugt weniger als 30 °C.

Der Wassergehalt des zu trocknenden Polymergels beträgt bei Eintritt in die Temperaturzone $T_n$ vorzugsweise mindestens 30 Gew.-%, besonders bevorzugt mindestens 40 Gew.-%, ganz besonders bevorzugt mindestens 50 Gew.-%, und vorzugsweise bis zu 70 Gew.-%, besonders bevorzugt bis zu 65 Gew.-%, ganz besonders bevorzugt bis zu 60 Gew.-%.

Der Wassergehalt des getrockneten Polymergels beträgt mindestens 3 Gew.-%, ganz besonders bevorzugt mindestens 5 Gew.-%, und bis zu 10 Gew.-%, besonders bevorzugt bis zu 9 Gew.-%, ganz besonders bevorzugt bis zu 8 Gew.-%.

Der Wassergehalt wird analog der von der EDANA (European Disposables and Nonwovens Association) empfohlenen Testmethode Nr. 430.2-02 "Moisture content" bestimmt.

In einer bevorzugten Ausführungsform wird dem Polymergel vor dem Trocknen ein Trennmittel zugesetzt. Vorzugsweise wird das Trennmittel unmittelbar vor dem Trocknen zugesetzt. Trennmittel vermindern das Zusammenkleben der Gelpartikel. Geeignete Trennmittel sind Tenside, beispielsweise mit einem HLB-Wert von weniger als 12, wie Sorbitanmonooleat, anorganische Pulver, wie pyrogene Kieselsäure, und organische Pulver, wie wasserabsorbierende Polymerpartikel. Der HLB-Wert ist ein Maß für die Wasser- bzw. Öl-Löslichkeit von Tensiden und kann nach üblichen Methoden bestimmt, beispielsweise gemäß den in "Surface Active Agents and Detergents", Band 2, Interscience Publishers, Inc., Seiten 479 ff, angegebenen Methoden, oder Tabellenwerken entnommen werden. Die Pulver weisen üblicherweise eine mittlere Partikelgröße von weniger als 300 $\mu$m, vorzugsweise weniger als 250 $\mu$m, besonders bevorzugt weniger als 200 $\mu$m, ganz besonders bevorzugt von weniger als 150 $\mu$m, auf. Geeignete wasserabsorbierende Polymerpartikel sind vorzugsweise Polymerpartikel, die während der Herstellung wasserabsorbierender Polymerpartikel anfallen und bei der Klassierung als Unterkorn abgetrennt werden. Die Partikelgröße kann beispielsweise nach der von der EDANA (European Disposables and Nonwovens Association) empfohlenen Testmethode Nr. 420.2-02 "Particle size distribution" bestimmt werden. Vorzugsweise werden getrocknete wasserabsorbierende Polymerpartikel mit einem Wassergehalt von weniger als 10 Gew.-%, vorzugsweise von weniger als 5 Gew.-%, besonders bevorzugt von weniger als 3 Gew.-%, verwendet.

Die Geschwindigkeit des die Polymergelschicht durchströmenden Gasstromes beträgt vorzugsweise mindestens 0,5 m/s, besonders bevorzugt mindestens 0,8 m/s, ganz besonders bevorzugt mindestens 1 m/s, und vorzugsweise bis zu 5 m/s, besonders bevorzugt bis zu 3 m/s, ganz besonders bevorzugt bis zu 2 m/s.

Das zu verwendende Gas unterliegt keiner Beschränkung. Zur Trocknung können Luft, Stickstoff oder andere unter den Trockenbedingungen inerte Gase eingesetzt werden. Luft ist bevorzugt.

Die Verweilzeit bei der Trocknung beträgt vorzugsweise mindestens 10 Minuten, besonders bevorzugt mindestes 15 Minuten, ganz besonders bevorzugt mindestens 20 Minuten, und vorzugsweise bis zu 100 Minuten, besonders bevorzugt bis zu 80 Minuten, ganz besonders bevorzugt bis zu 60 Minuten.

Die Trocknung wird vorzugsweise bei einem Druck durchgeführt, der gegenüber dem Atmosphärendruck vermindert ist, vorzugsweise um mindestens 0,5 mbar, besonders bevorzugt um mindestens 2 mbar, ganz besonders bevorzugt um mindestens 10 mbar.

Das für die vorliegende Erfindung bevorzugte Verfahren ist ein Förderbandverfahren (Bandtrockner). Der Bandtrockner ist ein konvektives Trocknungssystem für die besonders schonende Behandlung von durchlüftbaren Produkten. Das zu trocknende Produkt wird auf ein endloses, gasdurchlässiges Förderband gegeben und mittels eines erwärmten Gasstromes, vorzugsweise Luft, durchströmt.

Das Trocknungsgas wird im Kreis geführt, um beim mehrfachen Durchgang durch die Produktschicht eine möglichst hohe Aufsättigung zu erfahren. Ein gewisser Anteil des Trockengases, vorzugsweise mindestens 10 %, besonders bevorzugt mindestens 15 %, ganz besonders bevorzugt mindestens 20 %, und vorzugsweise bis zu 50 %, besonders bevorzugt bis zu 40 %, ganz besonders bevorzugt bis zu 30 %, der Gasmenge pro Durchgang, verlässt den Trockner als hoch aufgesättigter Brüden und führt die aus dem Produkt verdampfte Wassermenge ab.

Die Größe und Ausführung der Trockner richtet sich nach dem zu verarbeitenden Produkt, der Produktionskapazität und der Trocknungsaufgabe.

Der Bandtrockner kann als Einband-, Mehrband-, Mehrstufen-, oder Mehretagensystem ausgestattet werden. Bevorzugt ist für die vorliegende Erfindung der Betrieb eines Bandtrockners mit mindestens einem Band. Ganz besonders bevorzugt sind Einbandtrockner. Um die Bandtrocknung verfahrenstechnisch optimal durchzuführen, werden die Trocknungseigenschaften der wasserabsorbierenden Polymere in Abhängigkeit von den gewählten Prozessparametern individuell ermittelt. Die Lochgröße und Maschenweite des Bandes wird dem Produkt angepaßt. Auch bestimmte Oberflächenveredelungen, wie Elektropolieren oder Teflonisieren, sind möglich.

Zur optimalen Produktförderung können alle, dem Fachmann bekannten kettengeführten und kettenlosen Bandsysteme eingesetzt werden, wie beispielsweise Plattenbänder, Dünnblech- und Endlosplattenbänder, Kunststoff- und Metallgewebebänder.

Zur wirtschaftlichen Trocknung der wasserabsorbierenden Polymere wird die Gasführung im Trockner konsequent auf einen energieeffizienten Betrieb ausgelegt. Es sind verschiedene Gasführungskonzepte möglich, die Vorteile hinsichtlich Trocknungsverhalten und Energieausnutzung aufweisen. Energierückgewinnungssysteme können eingesetzt werden, um Wärme aus dem Abgasstrom zur Vorwärmung des zugeführten Frischgases zu nutzen.

Die Beheizung des Trockners kann direkt oder indirekt über die verschiedenen Heizmedien wie Dampf, Warmwasser, Rauchgase, Thermalöl oder Gas, vorzugsweise Dampf, erfolgen.

Eine Voraussetzung zur optimalen Trocknung ist die gleichmäßige Produktaufgabe. Diese kann gestaltet werden durch den Einsatz von schwenkbaren und oszillierenden Verteilbändern, Schwingrinnen oder Schnecken, Vibrationsrinnen oder Schwingförderern.

Das zu trocknende Polymergel wird vorzugsweise mittels eines Schwenkbandes auf das Band des Bandtrockners aufgebracht. Die Aufgabehöhe, d. h., der vertikale Abstand zwischen Schwenkband und Band, beträgt vorzugsweise mindestens 10 cm, besonders bevorzugt mindestens 20 cm, ganz besonders bevorzugt mindestens 30 cm, vorzugsweise bis zu 200 cm, besonders bevorzugt bis zu 120 cm, ganz besonders bevorzugt bis zu 40 cm.

Die Schichtdicke des zu trocknenden Polymergels auf dem Bandtrockner beträgt vorzugsweise mindestens 1 cm, besonders bevorzugt mindestens 2 cm, ganz besonders bevorzugt mindestens 4 cm, und vorzugsweise weniger als 10 cm, besonders bevorzugt weniger als 9 cm , ganz besonders bevorzugt weniger als 8 cm.

Die Bandgeschwindigkeit des Bandtrockners beträgt vorzugsweise mindestens 0,005 m/s, besonders bevorzugt mindestens 0,01 m/s, ganz besonders bevorzugt mindestens 0,015 m/s, und vorzugsweise bis zu 0,05 m/s, besonders bevorzugt bis zu 0,03 m/s, ganz besonders bevorzugt bis zu 0,025 m/s.

In einer bevorzugten Ausführungsform wird das zu trocknende Polymergel im vorderen Abschnitt des Bandtrockners von unten nach oben und im hinteren Abschnitt des Bandtrockners von oben nach unten von dem Gasstrom durchströmt, wobei die Strömungsumkehr bei einem Wassergehalt des Polymergels von vorzugsweise mindestens 15 Gew.-%, bevorzugt mindestens 20 Gew.-%, besonders bevorzugt mindestens 25 Gew.-%, ganz besonders bevorzugt mindestens 28 Gew.-%, und vorzugsweise höchstens 45 Gew.-%, bevorzugt höchstens 35 Gew.-%, besonders bevorzugt höchstens 32 Gew.-%, ganz besonders bevorzugt höchstens 30 Gew.-%, stattfindet.

Vorzugsweise ist die Gasgeschwindigkeit nach der Strömungsumkehr erhöht, vorzugsweise um mindestens 10 %, besonders bevorzugt um mindesten 30 %, ganz besonders um mindestens 40 %, und vorzugsweise um bis zu 100 %, besonders bevorzugt um bis zu 80 %, ganz besonders um bis zu 60 %.

Wird der Bandtrockner zumindest teilweise von unten nach oben durchströmt, so beträgt die Gasgeschwindigkeit vorzugsweise mindestens 5 %, besonders bevorzugt mindestens 8 %, ganz besonders bevorzugt mindestens 10 %, und vorzugsweise bis zu 30 %, besonders bevorzugt bis zu 25 %, ganz besonders bevorzugt bis zu 20 %, der Gasgeschwindigkeit, die notwendig ist um das Polymergel vom Band zu lösen.

Die Gas- bzw. Luftgeschwindigkeit, bei der sich die Polymergelschicht vom Band ablöst (Wirbelpunkt), kann experimentell bestimmt oder gemäß $v_{max} = \sqrt{\frac{\rho_B \times g \times \Delta h}{c_D}}$ berechnet werden, wobei $v_{max}$ die maximale Gas- bzw. Luftgeschwindigkeit ist, bei der sich das Polymergel vom Band ablöst $\rho_B$ das Schüttgewicht, g die Gravitationskonstante, $\Delta h$ der Druckverlust über die Polymergelschicht und $c_D$ der Gas- bzw. Luftwiderstandsbeiwert ist.

Geeignete Monomere a) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

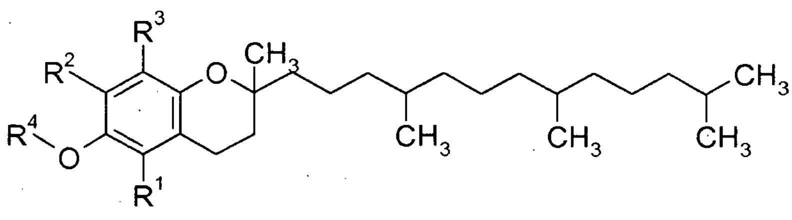

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 30 Gew.-ppm, besonders bevorzugt mindestens 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Polymerisationsinhibitoren können auch durch Absorption, beispielsweise an Aktivkohle, aus der Monomerlösung entfernt werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO- 90/15830 A1 und WO 2002/32962 A2 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP 343 427 A2 beschrieben sind. Weiterhin geeignete Vernetzer b) sind Pentaerythritoldi- Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Daher können die Polymerisationsinhibitoren vor der Polymerisation durch Inertisierung, d. h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten Grundpolymers sowie weitere geeignete hydrophile ethylenisch ungesättigte Monomere d) werden in DE 199 41 423 A1, EP 686 650 A1, WO 2001/45758 A1 und WO 2003/104300 A1 beschrieben.

Wasserabsorbierende Polymere werden üblicherweise durch Polymerisation einer wässrigen Monomerlösung und gegebenenfalls einer anschließenden Zerkleinerung des Polymergels erhalten. Geeignete Herstellverfahren sind in der Literatur beschrieben. Wasserabsorbierende Polymere können beispielsweise erhalten werden durch

Gelpolymerisation im Batchverfahren bzw. Rohrreaktor und anschließender Zerkleinerung im Fleischwolf, Extruder oder Kneter (EP 445 619 A2, DE 198 46 413 A1), Polymerisation im Kneter, wobei durch beispielsweise gegenläufige Rührwellen kontinuierlich zerkleinert wird (WO 2001/38402 A1),
Polymerisation auf dem Band und anschließende Zerkleinerung im Fleischwolf, Extruder oder Kneter (DE 38 25 366 A1, US 6,241,928),
Emulsionspolymerisation, wobei bereits Perlpolymerisate relativ enger Gelgrößenverteilung anfallen (EP 457 660 A1).

Die Umsetzung wird vorzugsweise in einem Kneter, wie beispielsweise in WO 2001/38402 A1 beschrieben, oder auf einem Bandreaktor, wie beispielsweise in EP 955 086 A2 beschrieben, durchgeführt.

Die Säuregruppen der erhaltenen Polymergele sind zu weniger als 55 mol-% neutralisiert, vorzugsweise zu 10 bis 55 mol-%, bevorzugt zu 20 bis 50 mol-%, besonders bevorzugt zu 25 bis 45 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium als Alkalimetalle besonders bevorzugt sind, ganz besonders bevorzugt jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23 °C vorliegen. In diesem Fall ist eine Dosierung als Stückgut oder Schmelze bei erhöhter Temperatur möglich.

Die Neutralisation kann nach der Polymerisation auf der Stufe des Polymergels durchgeführt werden. Es ist aber auch möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Die Monomerlösung kann durch Einmischen des Neutralisationsmittels neutralisiert werden. Das Polymergel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung auf den Endneutralisationsgrad ist bevorzugt.

Anschließend werden die erhaltenen wässrigen Polymergele gemäß dem oben beschriebenen erfindungsgemäßen Verfahren getrocknet.

Auf die weitere Behandlung des getrockneten Polymergels kommt es bei dem erfindungsgemäßen Verfahren nicht an. Das erfindungsgemäße Verfahren kann beispielsweise noch die Schritte Mahlung, Siebung und/oder Nachvernetzung umfassen.

Das getrocknete Polymergel wird vorzugsweise gemahlen und gesiebt, wobei zur Mahlung üblicherweise Walzenstühle, Hammermühlen, Stiftmühlen oder Schwingmühlen eingesetzt werden können. Die Partikelgröße des gesiebten, trockenen Polymergels beträgt vorzugsweise unter 1000 μm, besonders bevorzugt unter 900 μm, ganz besonders bevorzugt unter 800 μm, und vorzugsweise über 100 μm, besonders bevorzugt über 150 μm, ganz besonders bevorzugt über 200 μm.

Ganz besonders bevorzugt ist eine Partikelgröße (Siebschnitt) von 106 bis 850 μm. Die Partikelgröße wird gemäß der von der EDANA (European Disposables and Nonwovens Association) empfohlenen Testmethode Nr. 420.2-02 "Particle size distribution" bestimmt.

Die Grundpolymere werden vorzugsweise anschließend oberflächennachvernetzt. Hierzu geeignete Nachvernetzer sind Verbindungen, die Gruppen enthalten, die mit mindestens zwei Carboxylatgruppen des Polymergels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP 83 022 A2, EP 543 303 A1 und EP 937 736 A2 beschrieben, di- oder polyfunktionelle Alkohole, wie in DE 33 14 019 A1, DE 35 23 617 A1 und EP 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 A1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/31482 A1 Morpholin-2,3-dion und dessen Derivate als geeignete Oberflächennachvernetzer beschrieben.

Die Nachvernetzung wird üblicherweise so durchgeführt, dass eine Lösung des Oberflächennachvernetzers auf das Polymergel oder das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung

stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250 °C, bevorzugt bei 50 bis 200 °C, und besonders bevorzugt bei 50 bis 150 °C. Die bevorzugte Verweilzeit bei dieser Temperatur im Reaktionsmischer oder Trockner beträgt unter 30 Minuten, besonders bevorzugt unter 10 Minuten.

Ein weiterer Gegenstand der vorliegenden Erfindung sind wasserabsorbierende Polymerpartikel, enthaltend

i) mindestens ein einpolymerisiertes ethylenisch ungesättigtes, säuregruppentragendes Monomer, das zu weniger als 55 mol-% neutralisiert ist,
ii) mindestens einen einpolymerisierten Vernetzer,
iii) wahlweise ein oder mehrere einpolymerisierte mit den unter i) genannten Monomeren copolymerisierbare ethylenisch und/oder allylisch ungesättigte Monomere,
iv) wahlweise ein oder mehrere wasserlösliche Polymere, auf die die unter i) genannten Monomere zumindest teilweise aufgepfropft sind,

wobei die Polymerpartikel einen Wassergehalt von weniger als 10 Gew.-% und einen Hunter 60-Wert von mindestens 60 aufweisen, sowie der Hunter 60-Wert während einer Lagerung von einer Woche bei 70 °C und einer relativen Luftfeuchtigkeit von 90 % um weniger als 10 % des Ursprungswertes fällt.

Die Säuregruppen der wasserabsorbierenden Polymerpartikel sind vorzugsweise zu 10 bis 55 mol-%, bevorzugt zu 20 bis 50 mol-%, besonders bevorzugt zu 25 bis 45 mol-%, neutralisiert.

Die Farbmessung wird entsprechend dem CIELAB-Verfahren (Hunterlab, Band 8, Jahrgang 1996, Heft 7, Seiten 1 bis 4) durchgeführt. Dabei werden die Farben in über die Koordinaten L*, a* und b* eines dreidimensionalen Systems beschrieben. Dabei gibt L* die Helligkeit an, wobei L* = 0 schwarz und L* = 100 weiß bedeutet. Die Werte für a* und b* geben die Position der Farbe auf den Farbachsen rot/grün bzw. gelb/blau an, wobei +a* für rot, -a* für grün, +b* für gelb und -b* für blau steht.

Die Farbmessung entspricht dem Dreibereichsverfahren nach DIN 5033-6.

Der Hunter 60-Wert ist ein Maß für den Weißegrad von Oberflächen und ist definiert als L*-3b*, d. h., der Wert ist umso niedriger, je dunkler und je gelbstichiger eine Farbe ist.

Der Hunter 60-Wert beträgt vorzugsweise mindestens 65, besonders bevorzugt mindestens 68, ganz besonders bevorzugt mindestens 70.

Der Hunter 60-Wert fällt während einer Lagerung von einer Woche bei 70 °C und einer relativen Luftfeuchtigkeit von 90 % vorzugsweise um weniger als 12 %, besonders bevorzugt um weniger als 10 %, ganz besonders bevorzugt um weniger als 8 % des Ursprungswertes.

Der b*-Wert beträgt vorzugsweise weniger als 10, bevorzugt weniger als 7, besonders bevorzugt weniger als 5, ganz besonders bevorzugt weniger als 4.

Der b*-Wert steigt während einer Lagerung von einer Woche bei 70 °C und einer relativen Luftfeuchtigkeit von 90 % vorzugsweise um weniger als 75 %, besonders bevorzugt um weniger als 50 %, ganz besonders bevorzugt um weniger als 30 % des Ursprungswertes.

Der Wassergehalt der erfindungsgemäßen Polymerpartikel beträgt vorzugsweise mindestens 2 Gew.-%, besonders bevorzugt mindestens 3 Gew.-%, ganz besonders bevorzugt mindestens 5 Gew.-%, und vorzugsweise bis zu 10 Gew.-%, besonders bevorzugt bis zu 9 Gew.-%, ganz besonders bevorzugt bis zu 8 Gew.-%.

Die mittlere Partikelgröße der erfindungsgemäßen wasserabsorbierenden Polymerpartikel beträgt vorzugsweise 200 bis 700 μm, besonders bevorzugt von 300 bis 600 μm, ganz besonders von 300 bis 500 μm. Die mittlere Partikelgröße der Produktfraktion kann mittels der von der EDANA (European Disposables and Nonwovens Association) empfohlenen Testmethode Nr. 420.2-02 "Partikel size distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Vorzugsweise weisen weniger als 10 Gew.-%, besonders bevorzugt weniger als 5 Gew.-%, ganz besonders bevorzugt weniger als 2 Gew.-%, der erfindungsgemäßen Polymerpartikel eine Partikelgröße von weniger als 150 μm auf. Ein zu hoher Anteil kleiner Partikel erhöht zwar den Weißegrad, senkt aber die Durchlässigkeit der gequollenen Gelschicht für weitere Flüssigkeit.

Die erfindungsgemäßen wasserabsorbierenden Polymerpartikel weisen typischerweise eine Zentrifugenretentionskapazität (CRC) von mindestens 20 g/g, vorzugsweise mindestens 23 g/g, besonders bevorzugt mindestens 25 g/g, und üblicherweise von weniger als 100 g/g auf. Die Zentrifugenretentionskapazität (CRC) wird gemäß der von der EDANA (European Disposables and Nonwovens Association) empfohlenen Testmethode Nr. 441.2-02 "Centrifuge retention capacity" bestimmt.

Ein weiterer Gegenstand der vorliegenden Erfindung sind Hygieneartikel, insbesondere Windeln.

Zur Bestimmung der Güte werden die getrockneten wasserabsorbierenden Polymerpartikel mit den nachfolgend beschrieben Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

Wassergehalt

Der Wassergehalt der wasserabsorbierenden Polymerpartikel wird gemäß der von der EDANA (European Disposables and Nonwovens Association) empfohlenen Testmethode Nr. 430.2-02 "Moisture content" bestimmt.

Zentrifugenretentionskapazität (CRC Centrifuge Retention Capacity)

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

Farbmessung

Für Farbmessung wurde ein Hunterlab LabScan Colorimeter (Modell LS-5100) verwendet.

Beispiele:

Beispiele 1 bis 7

1235,1 g einer 37,3 gew.-%igen wässrigen Natriumacrylatlösung, 529,31 g Acrylsäure, 1182,77 g Wasser, 1,32g Harnstoff, 1,32 g 3-fach ethoxiliertes Glyzerintriacrylat wurden in ein 5.000 ml Becherglas eingewogen. Der Neutralisationsgrad betrug 40 mol-%.

Diese Lösung wurde dann 30 Minuten mittels einer Metallfritte mit 100l/h Stickstoff inertisiert. Zusätzlich wurde die Lösung gerührt (~100U/min). Während des Rührens wurde die Monomerlösung auf 10 °C gekühlt. Kurz vor der Zugabe der Initiatoren wurde das Kühlbad entfernt. Es wurden nacheinander 10,0 g einer 3 gew.-%igen wässrigen Lösung von 2,2'-Azobis-(2-amidinopropan)dihydrochlorid, 30,0 g einer 3 gew.-%igen wässrigen Lösung von Natriumpersulfat, 7,0 g einer 3 gew.-%igen wässrigen Lösung von Wasserstoffperoxid und 4,5 g einer 1 gew.-%igen wässrigen Lösung von Ascorbinsäure zugegeben. Nach dem Reaktionsstart wurden Rührer und Metallfritte aus der Reaktionslösung entfernt. Nach einer Reaktionszeit von 30 Minuten wurde das Gel entnommen und mit einem Fleischwolf mit Lochplatte (6 mm Lochdurchmesser) zerkleinert.

700 g des zerkleinerten Gels wird auf eine Blech mit Gitterboden gleichmäßig verteilt und für 75 Minuten in einem Umlufttrockenschrank (Heraeus UT 12, maximale Umluft) bei der in der Tabelle angegeben Temperatur getrocknet.

Nach dreimaligen Mahlen in einem Walzenstuhl (Gebr. Baumeister LRC 125/70, Spaltbreiten 1000 *m, 600 *m, 400 *m) wir das Polymer auf einen Siebschnitt zwischen 850 und 100 μm abgesiebt.

Von den erhaltenen wasserabsorbierenden Polymerpartikeln wurde die Zentrifugenretentionskapazität (CRC) bestimmt. Die Ergebnisse sind in Tabelle 1 zusammengefasst.

Tab. 1: Trocknung bei unterschiedlichen Temperaturen (Neutralisationsgrad von 40 mol-%)

| Beispiel | Temperatur | Zentrifugenretentionskapazität (CRC) |
|---|---|---|
| 1*) | 160 °C | 29,5 g/g |
| 2*) | 165 °C | 29,4 g/g |
| 3*) | 170 °C | 26,9 g/g |
| 4*) | 175 °C | 24,2 g/g |
| 5*) | 180 °C | 24,0 g/g |
| 6*) | 185 °C | 22,9 g/g |
| 7*) | 190 °C | 20,8 g/g |
| *) nicht erfindungsgemäß | | |

Mit steigender Trocknungstemperatur wird die Zentrifugenretentionskapazität (CRC) immer schlechter.

Beispiele 8 bis 14

2478,79 g einer 37,3 gew.-%igen wässrigen Natriumacrylatlösung, 275,41 g Acrylsäure, 192,91 g Wasser, 4,13 g 3-fach ethoxiliertes Glyzerintriacrylat wurden in ein 5.000 ml Becherglas eingewogen. Der Neutralisationsgrad betrug 72 mol-%. Diese Lösung wurde dann 30 Minuten mittels einer Metallfritte mit 100l/h Stickstoff inertisiert. Zusätzlich wurde die Lösung gerührt (~100U/min). Während des Rührens wurde die Monomerlösung auf 10 °C gekühlt. Kurz vor der Zugabe der Initiatoren wurde das Kühlbad entfernt. Es wurden nacheinander 10,0 g einer 3 gew.-%igen wässrigen Lösung von 2,2'-Azobis-(2-amidinopropan)dihydrochlorid, 30,0 g einer 3 gew.-%igen wässrigen Lösung von Natriumpersulfat, 7,0 g einer 3 gew.-%igen wässrigen Lösung von Wasserstoffperoxid und 4,5 g einer 1 gew.-%igen wässrigen Lösung von Ascorbinsäure zugegeben. Nach dem Reaktionsstart wurden Rührer und Metallfritte aus der Reaktionslösung entfernt. Nach einer Reaktionszeit von 30 Minuten wurde das Gel entnommen und mit einem Fleischwolf mit Lochplatte (6 mm Lochdurchmesser) zerkleinert.

700 g des zerkleinerten Gels wird auf ein Blech mit Gitterboden gleichmäßig verteilt und für 75 Minuten in einem Umlufttrockenschrank (Heraeus UT 12, maximale Umluft) bei der in der Tabelle angegeben Temperatur getrocknet.

Nach dreimaligen Mahlen in einem Walzenstuhl (Gebr. Baumeister LRC 125/70, Spaltbreiten 1000 μm, 600 μm, 400 μm) wir das Polymer auf einen Siebschnitt zwischen 850 und 100 μm abgesiebt.

Von den erhaltenen wasserabsorbierenden Polymerpartikeln wurde die Zentrifugenretentionskapazität (CRC) bestimmt. Die Ergebnisse sind in Tabelle 2 zusammengefasst.

Tab. 2: Trocknung bei unterschiedlichen Temperaturen (Neutralisationsgrad von 70 mol-%)

| Beispiel | Temperatur | Zentrifugenretentionskapazität (CRC) |
|---|---|---|
| 8*) | 160 °C | 33,1 g/g |
| 9*) | 165 °C | 33,8 g/g |
| 10*) | 170 °C | 34,6 g/g |
| 11*) | 175 °C | 36,2 g/g |
| 12*) | 180 °C | 37,2 g/g |
| 13*) | 185 °C | 38,9 g/g |
| 14*) | 190 °C | 38,8 g/g |
| *) nicht erfindungsgemäß | | |

Mit steigender Trocknungstemperatur steigt ebenfalls die Zentrifugenretentionskapazität (CRC). Dieses Verhalten ist genau das Gegenteil von dem, was mit Polymergelen mit einem Neutralisationsgrad von 40 mol-% gefunden wurde.

Polymergele mit niedrigem Neutralisationsgrad müssen daher anders getrocknet werden als Polymergele mit

hohem Neutralisationsgrad.

Beispiele 15 bis 24

117,62 g Acrylsäure, 223,05 g Wasser und 52,28 g einer 50 gew.-%igen wässrigen Natriumhydroxidlösung wurden so gemischt, dass die Temperatur nicht über 35 °C stieg. Anschließend wurden 0,18 g Harnstoff und 0,18 g 3-fach ethoxiliertes Glyzerintriacrylat in ein 600 ml Becherglas eingewogen. Der Neutralisationsgrad betrug 40 mol-%. Diese Lösung wurde dann 30 Minuten mittels einer Metallfritte mit 100l/h Stickstoff inertisiert. Zusätzlich wurde die Lösung gerührt (~100U/min). Während des Rührens wurde die Monomerlösung auf 10 °C gekühlt. Kurz vor der Zugabe der Initiatoren wurde das Kühlbad entfernt. Es wurden nacheinander 0,16 g Sorbitanmonolaurat, 1,33 g einer 3 gew.-%igen wässrigen Lösung von 2,2'-Azobis-(2-amidinopropan)dihydrochlorid, 4,0 g einer 3 gew.-%igen wässrigen Lösung von Natriumpersulfat, 0,93 g einer 3 gew.-%igen wässrigen Lösung von Wasserstoffperoxid und 0,6 g einer 1 gew.-%igen wässrigen Lösung von Ascorbinsäure zugegeben. Nach dem Reaktionsstart wurden Rührer und Metallfritte aus der Reaktionslösung entfernt. Nach einer Reaktionszeit von 30 Minuten wurde das Gel entnommen und mit einem Fleischwolf mit Lochplatte (6 mm Lochdurchmesser) zerkleinert.

300 g des zerkleinerten Gels wurden in einen zylindrischen Behälter mit Gitterboden (Maschenweite der Bodenplatte: 350 µm, Höhe: 11 cm, Durchmesser: 10 cm) überführt und mit erwärmter, nicht befeuchteter Umgebungsluft (Luftgeschwindigkeit: 1,0 m/s) die Zeit $t_1$ bei der Temperatur $T_1$ und ggf. zusätzlich die Zeit $t_2$ bei der Temperatur $T_2$ getrocknet. Die Einstellungen sind in der Tabelle 3 zusammengefasst.

Nach dreimaligen Mahlen in einem Walzenstuhl (Gebr. Baumeister LRC 125/70, Spaltbreiten 1000 µm, 600 µm, 400 µm) wurde das Polymer auf einen Siebschnitt zwischen 850 und 100 µm abgesiebt.

Je 2,5 g der Proben wurden in eine kleine Kunststoffpetrischale (Höhe: 1,2 cm, Innendurchmesser: 3,5 cm) überführt und mit einem Macbeth Color-Eye 2180 (Equation: CIELab, Illuminants: D65, Observer: 10°, CMC Ratio Lightness (I): 2., CMC Ratio Chromaticity (c): 1., Chromatic Wavelength: Auto, Block Sizes A1: 55,00, Block Sizes A2: 66,00, Block Sizes A3: 77,00) der Hunter 60-Wert (HC 60) und der b*-Wert bestimmt. Diese Messung wurde nach einer Lagerung im Klimaschrank (90% rel. Luftfeuchte, 70 °C) für 7 bzw. 14 Tagen wiederholt.

Aus den Werten wurde die Änderung nach 7 Tagen gegenüber dem Anfangswert berechnet.

Die Ergebnisse sind in der Tabelle 3 zusammengefasst.

Tab. 3: Trocknung mit und ohne Temperaturprofil

| Beispiel | $t_1$ [min] | $T_1$ | $t_2$ [min] | $T_2$ | Wassergehalt | CRC [g/g] | ΔHC 60 | Δb* |
|---|---|---|---|---|---|---|---|---|
| 15**) | 25 | 150 °C | | | 3,4 Gew.-% | 32,2 | -18 % | +81 % |
| 16**) | 25 | 160 °C | | | 3,2 Gew.-% | 30,2 | -20 % | +72 % |
| 17**) | 25 | 165 °C | | | 2,4 Gew.-% | 28,6 | -18 % | +63 % |
| 18**) | 25 | 170 °C | | | 1,7 Gew.-% | 21,7 | -18 % | +58 % |
| 19**) | 10 | 200 °C | 15 | 170 °C | 2,9 Gew.-% | 31,4 | -24 % | +109 % |
| 20**) | 10 | 200 °C | 20 | 170 °C | 2,0 Gew.-% | 31,1 | -26 % | +134 % |
| 21 | 10 | 165 °C | 15 | 150 °C | 4,7 Gew.-% | 40.3 | -8 % | +70 % |
| 22 | 10 | 170 °C | 1,5 | 150 °C | 3,6 Gew.-% | 38,3 | -9 % | +57 % |
| 23***) | 15 | 165 °C | 10 | 150 °C | 2,8 Gew.-% | 31,0 | -7 % | +52 % |
| 24***) | 15 | 170 °C | 10 | 150 °C | 2,7 Gew.-% | 31,3 | -8 % | +25 % |
| **). Vergleichsbeispiele<br>***) Beispiel fällt nicht unter Anspruch 1 | | | | | | | | |

Tab. 3: Trocknung mit und ohne Temperaturprofil (Fortsetzung)

| Beispiel | sofort nach der Trocknung | | nach einer Lagerung von einer Woche | | nach einer Lagerung von zwei Wochen | |
|---|---|---|---|---|---|---|
| | HC 60 | b* | HC 60 | b* | HC 60 | b* |
| 15**) | 71,402 | 4,485 | 58,426 | 8,124 | 34,496 | 12,845 |

(fortgesetzt)

| Beispiel | sofort nach der Trocknung | | nach einer Lagerung von einer Woche | | nach einer Lagerung von zwei Wochen | |
|---|---|---|---|---|---|---|
| | HC 60 | b* | HC 60 | b* | HC 60 | b* |
| 16**) | 70,857 | 4,907 | 56,987 | 8,425 | 37,302 | 13,565 |
| 17**) | 70,569 | 5,067 | 57,945 | 8,269 | 38,846 | 13,645 |
| 18**) | 70,391 | 5,139 | 57,846 | 8,106 | 38,548 | 13,561 |
| 19**) | 73,805 | 3,828 | 56,328 | 7,984 | 39,027 | 13,000 |
| 20**) | 73,273 | 4,159 | 54,421 | 9,726 | 33,677 | 14,424 |
| 21 | 71,947 | 3,973 | 65,984 | 6,735 | 49,057 | 10,177 |
| 22 | 70,539 | 4,363 | 63,845 | 6,845 | 48,687 | 10,994 |
| 23***) | 70,335 | 4,595 | 65,240 | 6,963 | 44,896 | 10,302 |
| 24***) | 69,790 | 4,679 | 64,258 | 5,870 | 45,364 | 11,188 |
| **) Vergleichsbeispiele<br>***) Beispiel fällt nicht unter Anspruch 1 | | | | | | |

**Patentansprüche**

**1.** Verfahren zur Herstellung wasserabsorbierender Polymerpartikel durch Polymerisation einer Monomerlösung, enthaltend

a) mindestens ein ethylenisch ungesättigtes, säuregruppentragendes Monomer, das zu weniger als 55 mol-% neutralisiert ist,
b) mindestens einen Vernetzer,
c) wahlweise ein oder mehrere mit den unter a) genannten Monomeren copolymerisierbare ethylenisch und/oder allylisch ungesättigte Monomere und
d) wahlweise ein oder mehrere wasserlösliche Polymere,

wobei die Monomerlösung polymerisiert und das erhaltene Polymergel mittels eines erwärmten Gasstromes getrocknet wird, **dadurch gekennzeichnet, dass** die Trocknung in mindestens zwei Temperaturzonen durchgeführt wird, wobei die Gaseingangstemperaturen der mindestens zwei Temperaturzonen die Bedingung $T_n$ größer $T_{n+a}$ erfüllen, wobei die Indizes n und a jeweils eine ganze Zahl größer 0 bedeuten, sowie die Gaseingangstemperatur in der Temperaturzone $T_n$ weniger als 175 °C und die Temperaturdifferenz der Gaseingangstemperaturen der Temperaturzonen $T_n$ und $T_{n+a}$ mehr als 10 °C beträgt und das Polymergel nach der Trocknung einen Wassergehalt von 3 bis 10 Gew.-% aufweist.

**2.** Verfahren gemäß Anspruch 1, **dadurch gekennzeichnet, dass** der Wassergehalt des Polymergels bei Eintritt in die Temperaturzone $T_n$ mindestens 30 Gew.-% beträgt.

**3.** Verfahren gemäß Anspruch 1 oder 2, **dadurch gekennzeichnet, dass** das Polymergel ein Trennmittel enthält.

**4.** Verfahren gemäß einem der Ansprüche 1 bis 3, **dadurch gekennzeichnet, dass** die Schichtdicke des zu trocknenden Polymergels weniger als 10 cm beträgt.

**5.** Verfahren gemäß einem der Ansprüche 1 bis 4, **dadurch gekennzeichnet, dass** die Geschwindigkeit des die Polymergelschicht durchströmenden Gasstroms von 0,5 bis 5 m/s beträgt.

**6.** Verfahren gemäß einem der Ansprüche 1 bis 5, **dadurch gekennzeichnet, dass** die Verweilzeit des Polymergels im Trockner von 10 bis 120 Minuten beträgt.

**7.** Verfahren gemäß einem der Ansprüche 1 bis 6, **dadurch gekennzeichnet, dass** der Druck bei der Trocknung

niedriger ist als Atmosphärendruck.

8. Verfahren gemäß einem der Ansprüche 1 bis 7, **dadurch gekennzeichnet, dass** die Trocknung in einem Bandtrockner durchgeführt wird.

9. Verfahren gemäß einem der Ansprüche 1 bis 8, **dadurch gekennzeichnet, dass** das Polymergel nach der Trocknung einen Wassergehalt von 3 bis 8 Gew.-% aufweist.

10. Wasserabsorbierende Polymerpartikel, enthaltend

i) mindestens ein einpolymerisiertes ethylenisch ungesättigtes, säuregruppentragendes Monomer, das zu weniger als 55 mol-% neutralisiert ist,
ii) mindestens einen einpolymerisierten Vernetzer,
iii) wahlweise ein oder mehrere einpolymerisierte mit den unter i) genannten Monomeren copolymerisierbare ethylenisch und/oder allylisch ungesättigte Monomere,
iv) wahlweise ein oder mehrere wasserlösliche Polymere, auf die die unter i) genannten Monomere zumindest teilweise aufgepfropft sind,

wobei die Polymerpartikel einen Wassergehalt von 3 bis 10 Gew.-% und einen Hunter 60-Wert von mindestens 60 aufweisen, sowie der Hunter 60-Wert während einer Lagerung von einer Woche bei 70 °C und einer relativen Luftfeuchtigkeit von 90 % um weniger als 15 % des Ursprungswertes fällt.

11. Polymerpartikel, gemäß Anspruch 10, **dadurch gekennzeichnet, dass** die Polymerpartikel einen b*-Wert von weniger als 10 aufweisen, sowie der b*-Wert während einer Lagerung von einer Woche bei 70 °C und einer relativen Luftfeuchtigkeit von 90 % um weniger als 75 % des Ursprungswertes steigt.

12. Polymerpartikel, gemäß Anspruch 10 oder 11, **dadurch gekennzeichnet, dass** der Anteil an Partikeln mit einem Durchmesser kleiner 150 µm weniger als 10 Gew.-% beträgt.

13. Polymerpartikel, gemäß einem der Ansprüche 10 bis 12, **dadurch gekennzeichnet, dass** die Polymerpartikel eine Zentrifugenretentionskapazität (CRC) von mindestens 20 g/g aufweisen.

14. Polymerpartikel, gemäß einem der Ansprüche 10 bis 13, **dadurch gekennzeichnet, dass** die Polymerpartikel eine mittlere Partikelgröße im Bereich von 300 bis 600 µm aufweisen.

15. Hygieneartikel, enthaltend wasserabsorbierende Polymerpartikel gemäß einem der Ansprüche 10 bis 14.

## Claims

1. A process for producing water-absorbing polymeric particles by polymerization of a monomer solution comprising

a) at least one ethylenically unsaturated acid-functional monomer less than 55 mol% neutralized,
b) at least one crosslinker,
c) selectively one or more ethylenically and/or allylically unsaturated monomers copolymerizable with the monomers mentioned under a), and
d) selectively one or more water-soluble polymers,

the monomer solution being polymerized and the polymeric gel obtained being dried by means of a heated gas stream, which comprises effecting the drying in two or more temperature zones for which the gas inlet temperatures satisfy the condition $T_n$ greater than $T_{n+a}$, where the indices n and a are each a whole number greater than 0, the gas inlet temperature in the temperature zone $T_n$ being less than 175°C and the temperature difference of the gas inlet temperatures of the temperature zones $T_n$ and $T_{n+a}$ being more than 10°C and the polymeric gel having a water content in the range from 3% to 10% by weight after drying.

2. The process according to claim 1 wherein the water content of the polymeric gel on entry into the temperature zone $T_n$ is at least 30% by weight.

3. The process according to claim 1 or 2 wherein the polymeric gel comprises a release agent.

4. The process according to any one of the claims 1 to 3 wherein the layer thickness of the polymeric gel to be dried is less than 10 cm.

5. The process according to any one of the claims 1 to 4 wherein the velocity of the gas stream flowing through the polymeric gel layer is in the range from 0.5 to 5 m/s.

6. The process according to any one of the claims 1 to 5 wherein the residence time of the polymeric gel in the dryer is in the range from 10 minutes to 120 minutes.

7. The process according to any one of the claims 1 to 6 wherein the pressure prevailing during the drying is less than the atmospheric pressure.

8. The process according to any one of the claims 1 to 7 wherein the drying is effected in a belt dryer.

9. The process according to any one of the claims 1 to 8 wherein the polymeric gel has a water content in the range from 3% to 8% by weight after drying.

10. Water-absorbing polymeric particles comprising

i) at least one interpolymerized ethylenically unsaturated acid-functional monomer less than 55 mol% neutralized,
ii) at least one interpolymerized crosslinker,
iii) selectively one or more interpolymerized ethylenically and/or allylically unsaturated monomers copolymerizable with the monomers mentioned under i),
iv) selectively one or more water-soluble polymers grafted at least partly with the monomers mentioned under i),

and having a water content in the range from 3% to 10% by weight and a Hunter 60 value of at least 60, the Hunter 60 value decreasing by less than 15% of the initial value during storage for one week at 70°C and a relative humidity of 90%.

11. The polymeric particles according to claim 10 which exhibit a b* value of less than 10 which rises by less than 75% of the initial value during storage for one week at 70°C and a relative humidity of 90%.

12. The polymeric particles according to claim 10 or 11 wherein the fraction of particles less than 150 μm in diameter is less than 10% by weight.

13. The polymeric particles according to any one of the claims 10 to 12 which exhibit a Centrifuge Retention Capacity (CRC) of at least 20 g/g.

14. The polymeric particles according to any one of the claims 10 to 13 which have a median particle size in the range from 300 to 600 μm.

15. A hygiene article comprising water-absorbing polymeric particles according to any one of the claims 10 to 14.

**Revendications**

1. Procédé pour la production de particules de polymère absorbant l'eau, par polymérisation d'une solution de monomères, contenant

a) au moins un monomère à insaturation éthylénique, contenant des groupes acides, qui est neutralisé à raison de moins de 55 % en moles,
b) au moins un agent de réticulation,
c) en option un ou plusieurs monomères à insaturation éthylénique et/ou allylique, copolymérisables avec les monomères nommés en a) et
d) en option un ou plusieurs polymères hydrosolubles,

dans lequel on polymérise la solution de monomères et le gel de polymère obtenu est séché au moyen d'un courant de gaz chauffé, **caractérisé en ce qu'**on effectue le chauffage dans au moins deux zones de température, les températures d'entrée de gaz desdites au moins deux zones de température remplissant la condition $T_n$ supérieure à $T_{n+a}$, les indices n et a représentant chacun un nombre entier plus grand que 0, ainsi que la température d'entrée de gaz dans la zone de température $T_n$ étant inférieure à 175 °C et la différence de température des températures d'entrée de gaz des zones de température $T_n$ et $T_{n+a}$ étant de plus de 10 °C et le gel de polymère après le séchage présentant une teneur en eau de 3 à 10 % en poids.

**2.** Procédé selon la revendication 1, **caractérisé en ce que** la teneur en eau du gel de polymère à l'entrée dans la zone de température $T_n$ est d'au moins 30 % en poids.

**3.** Procédé selon la revendication 1 ou 2, **caractérisé en ce que** le gel de polymère contient un agent de démoulage.

**4.** Procédé selon l'une quelconque des revendications 1 à 3, **caractérisé en ce que** l'épaisseur de couche du gel de polymère à sécher est inférieure à 10 cm.

**5.** Procédé selon l'une quelconque des revendications 1 à 4, **caractérisé en ce que** la vitesse du courant de gaz traversant la couche de polymère vaut de 0,5 à 5 m/s.

**6.** Procédé selon l'une quelconque des revendications 1 à 5, **caractérisé en ce que** le temps de séjour du gel de polymère dans le sécheur va de 10 à 120 minutes.

**7.** Procédé selon l'une quelconque des revendications 1 à 6, **caractérisé en ce que** la pression lors du chauffage est inférieure à la pression atmosphérique.

**8.** Procédé selon l'une quelconque des revendications 1 à 7, **caractérisé en ce que** le séchage est effectué dans un sécheur à bande.

**9.** Procédé selon l'une quelconque des revendications 1 à 8, **caractérisé en ce que** le gel de polymère présente après le séchage une teneur en eau de 3 à 8 % en poids.

**10.** Particules de polymère absorbant l'eau, contenant

i) au moins un monomère à insaturation éthylénique, contenant des groupes acides, qui est neutralisé à raison de moins de 55 % en moles, incorporé par polymérisation,
ii) au moins un agent de réticulation incorporé par polymérisation,
iii) en option un ou plusieurs monomères à insaturation éthylénique et/ou allylique, copolymérisables avec les monomères nommés en i), incorporés par polymérisation,
iv) en option un ou plusieurs polymères hydrosolubles, sur lesquels sont greffés au moins en partie les monomères nommés en i),

les particules de polymère présentant une teneur en eau de 3 à 10 % en poids et une valeur Hunter 60 d'au moins 60, et la valeur Hunter 60 diminuant de moins de 15 % de la valeur initiale pendant un stockage d'une semaine à 70 °C et à une humidité relative de l'air de 90 %.

**11.** Particules de polymère selon la revendication 10, **caractérisées en ce que** les particules de polymère présentent une valeur b* de moins de 10, et la valeur b* augmentant de moins de 75 % de la valeur initiale pendant un stockage d'une semaine à 70 °C et à une humidité relative de l'air de 90 %.

**12.** Particules de polymère selon la revendication 10 ou 11, **caractérisées en ce que** la proportion de particules ayant un diamètre inférieur à 150 μm est de moins de 10 % en poids.

**13.** Particules de polymère selon l'une quelconque des revendications 10 à 12, **caractérisées en ce que** les particules de polymère présentent une capacité de rétention centrifuge (CRC) d'au moins 20 g/g.

**14.** Particules de polymère selon l'une quelconque des revendications 10 à 13, **caractérisées en ce que** les particules de polymère présentent une taille moyenne de particule dans la plage de 300 à 600 μm.

15. Article d'hygiène, contenant des particules de polymère absorbant l'eau selon l'une quelconque des revendications 10 à 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 289338 A2
- EP 1002806 A1
- DE 102005014291
- EP 530438 A1
- EP 547847 A1
- EP 559476 A1
- EP 632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- EP 343427 A2
- DE 19941423 A1
- EP 686650 A1
- WO 200145758 A1
- EP 445619 A2
- DE 19846413 A1
- WO 200138402 A1
- DE 3825366 A1
- US 6241928 B
- EP 457660 A1
- EP 955086 A2
- EP 83022 A2
- EP 543303 A1
- EP 937736 A2
- DE 3314019 A1
- DE 3523617 A1
- EP 450922 A2
- DE 10204938 A1
- US 6239230 B
- DE 4020780 C1
- DE 19807502 A1
- DE 19807992 A1
- DE 19854573 A1
- DE 19854574 A1
- DE 10204937 A1
- DE 10334584 A1
- EP 1199327 A2
- WO 200331482 A1


### In der Beschreibung aufgeführte Nicht-Patentliteratur


- **F.L. BUCHHOLZ ; A.T. GRAHAM.** Modern Superabsorbent Polymer Technology. Wiley-VCH, 1998
- Ullmann's Encyclopedia of Industrial Chemistry. vol. 35, 73-103
- **F.L. BUCHHOLZ ; A.T. GRAHAM.** Modern Superabsorbent Polymer Technology. Wiley-VCH, 1998, 87-93
- Surface Active Agents and Detergents. Interscience Publishers, Inc, vol. 2, 479 ff
- *Hunterlab,* 1996, vol. 8, 1-4